**Europäisches Patentamt**

**European Patent Office**

**Office européen des brevets**

(19)

(11) Veröffentlichungsnummer: **0 053 692**
**B1**

# EUROPÄISCHE PATENTSCHRIFT

(12)

(45) Veröffentlichungstag der Patentschrift:
**12.10.83**

(51) Int. Cl.³: **C 12 Q 1/60**, G 01 N 33/92

(21) Anmeldenummer: **81108753.5**

(22) Anmeldetag: **22.10.81**

(54) **Verfahren und Reagenz zur Bestimmung von Cholesterin.**

(30) Priorität: **08.12.80 DE 3046241**

(43) Veröffentlichungstag der Anmeldung:
**16.06.82 Patentblatt 82/24**

(45) Bekanntmachung des Hinweises auf die Patenterteilung:
**12.10.83 Patentblatt 83/41**

(84) Benannte Vertragsstaaten:
**AT BE CH DE FR GB IT LI LU NL SE**

(56) Entgegenhaltungen:
**EP-A-0 004 857**
**EP-A-0 016 946**
**EP-A-0 021 311**
**GB-A-1 479 994**
**US-A-4 226 713**

**CHEMICAL ABSTRACTS, Band 87, Nr. 11,**
**12. September 1977, Seite 257, Nr. 80812a**
**Columbus, Ohio, U.S.A.,**
**T. OTANI et al.: "Determination of total and free**
**cholesterol by using cholesterol oxidase from**
**Streptomyces"**

(73) Patentinhaber: **BOEHRINGER MANNHEIM GMBH,**
**Patentabteilung, Abt. E Sandhofer**
**Strasse 112-132 Postfach 31 01 20,**
**D-6800 Mannheim 31 Waldhof (DE)**

(72) Erfinder: **Deeg, Rolf, Dr., Seeseitener Strasse 18,**
**D-8124 Seehaupt (DE)**
Erfinder: **Schlumberger, Helmut,**
**Kaiser-Heinrich-Strasse 23, D-8121 Polling (DE)**
Erfinder: **Ziegenhorn, Joachim, Dr., Ina-Seidel-Weg 1,**
**D-8130 Starnberg (DE)**

(74) Vertreter: **Weickmann, Heinrich, Dipl.-Ing. et al,**
**Patentanwälte Dipl.-Ing. H. Weickmann Dipl.-Phys.Dr. K.**
**Fincke Dipl.-Ing. F.A. Weickmann Dipl.-Chem. B. Huber**
**Dr.-Ing. H. Liska Möhlstrasse 22,**
**D-8000 München 86 (DE)**

Verfahren und Reagenz zur Bestimmung von Cholesterin

Die Erfindung betrifft ein Verfahren und ein Reagenz zur Bestimmung von Cholesterin unter Verwendung von Cholesterinoxidase.

Es ist bekannt, Cholesterin, gegebenenfalls nach vorheriger Freisetzung aus seinen Estern durch chemische oder enzymatische Verseifung, mittels Cholesterinoxidase in Gegenwart von molekularem Sauerstoff zu Cholestenon und $H_2O_2$ umzusetzen und diese quantitativ ablaufende Reaktion zur Bestimmung des Cholesterins zu benutzen. Hierbei wird entweder gebildetes Cholestenon bzw. $H_2O_2$ oder verbrauchter Sauerstoff gemessen. Dieses enzymatische Cholesterin-Bestimmungsverfahren hat zwar aufgrund seiner Spezifität gegenüber den früher gebräuchlichen chemischen Bestimmungsmethoden einen erheblichen Fortschritt gebracht, jedoch eignet sich das Verfahren bisher nicht zur raschen und praktikablen kinetischen Durchführung. Aufgrund des niedrigen $K_M$-Werts der Cholesterinoxidase, die den spezifischsten Teilschritt der Reaktionsfolge katalysiert, verläuft die Reaktion in dem für die Cholesterinbestimmung interessanten Konzentrationsbereich bis 13 mMol/l nicht nach erster bzw. pseudoerster Ordnung. Ein derartiger Reaktionsverlauf ist jedoch Voraussetzung für eine rasche, praktikable, keinen Probenleerwert erfordernde kinetische Bestimmungsmethode, die eine wesentliche Verkürzung des Zeitbedarfs pro Einzelanalyse gegenüber den bisher erforderlichen Endpunkt- bzw. kinetischen Methoden ermöglichen würde. Dabei lag der Zeitbedarf für die bisherigen photometrischen Verfahren zwischen 10 und 6 Minuten; angestrebt werden bei kinetischen Methoden Analysenzeiten von 1 bis 3 Minuten. Die Analysenautomaten der neuen Generation sind für einen hohen Probendurchsatz konzipiert und gestatten nur kurze Inkubationszeiten, die mit den bisherigen Verfahren, im Rahmen des Cholesterinnachweises, nicht erzielt werden können. Infolgedessen können die heute üblichen Analysenautomaten mit hoher Analysenfrequenz nicht voll ausgelastet werden.

Der Erfindung liegt daher die Aufgabe zugrunde, ein kinetisches Bestimmungsverfahren für die Cholesterinoxidase-Reaktion zu entwickeln, bei dem diese nach pseudoerster Ordnung abläuft.

Es ist bekannt, dass es in vielen Fällen möglich ist, einen derartigen Reaktionsverlauf bei zu niedrigen $K_M$-Werten der beteiligten Enzyme durch künstliche $K_M$-Werterhöhung zu erzielen. Aus der Theorie von Michaelis und Menten folgt, dass enzymkatalysierte Einsubstrat-Reaktionen dann über einen weiten Konzentrationsbereich nach erster Ordnung ablaufen, wenn die Michaelis-Konstante des Enzyms sehr viel grösser ist als die maximale Substratkonzentration. Da die bisher bekannten Cholesterinoxidasen aus verschiedenen Mikroorganismengattungen, wie Nocardia, Brevibacterium oder Streptomyceten $K_M$-Werte

in der Grössenordnung von $10^{-4}$ bis $10^{-5}$ mol/l aufweisen, lassen sich hier nur geringe Cholesterinkonzentrationen kinetisch messen. Versuche, durch Zusatz eines kompetitiven Inhibitors den $K_M$-Wert der Cholesterinoxidase in an sich bekannter Weise künstlich zu erhöhen, erwiesen sich als erfolglos.

Überraschenderweise wurde jedoch gefunden, dass es möglich ist, bei Auswahl einer Cholesterinoxidase bestimmer Herkunft und eines bestimmten Phenolderivats einen Reaktionsablauf nach pseudoerster Ordnung zu erzielen.

Das erfindungsgemässe Verfahren zur Bestimmung von Cholesterin in freier oder gebundener Form mittels Cholesterinoxidase und gegebenenfalls Cholesterinesterase durch Messung des Sauerstoffverbrauchs, des gebildeten Wasserstoffperoxids oder des gebildeten Cholestenons ist daher dadurch gekennzeichnet, dass die Bestimmung kinetisch durchgeführt wird unter Verwendung einer Cholesterinoxidase, die aus einem Mikroorganismus der Gattung Streptomyces gewonnen wurde und unter Zusatz von 3,4-Dichlorphenol.

Weder mit den bisher für die Cholesterinbestimmung gebräuchlichen Cholesterinoxidasen aus Nocardia oder Brevibacterium noch mit den als kompetitiven Hemmstoffen in erster Linie in Betracht kommenden Cholesterin ähnlichen Steroiden oder Gallensäuren konnte ein derartiger, die kinetische Bestimmung erst ermöglichender Reaktionsverlauf erzielt werden. Auch die anderen Isomeren des Dichlorphenols haben keinen oder nur einen ungenügenden Einfluss auf den Reaktionsverlauf. Es wird daher angenommen, dass zwischen dem 3,4-Dichlorphenol und dem Enzym aus Mikroorganismen der Gattung Streptomyces eine besondere Wechselwirkung auftritt.

Die Gewinnung von Cholesterinoxidase aus Streptomyceten wird in der DE-OS 29 24 875.7 beschrieben. Beispiele für geeignete Stämme sind Streptomyces griseofuscus DSM 40191, Streptomyces hygroscopicus DSM 40771 und Streptomyces acidomyceticus DSM 40798.

Der angestrebte Reaktionsablauf erster Ordnung lässt sich bei weit variierenden Konzentrationen von 3,4-Dichlorphenol und Cholesterinoxidase erzielen. Bevorzugt verwendet man 0,5 bis 10 mmol/ 3,4-Dichlorphenol und 0,1 bis $20 \times 10^3$ U/l Cholesterinoxidase aus Streptomyces. Die Durchführung des Verfahrens der Erfindung erfolgte im übrigen in der für kinetische Bestimmungen üblichen Weise. Vorzugsweise werden wenigstens zwei Messungen in einem vorher bestimmten Zeitintervall durchgeführt. Die Bestimmung kann im ganzen pH-Wertbereich, bei welchem die Cholesterinoxidase aus Streptomyces aktiv ist, vorgenommen werden, vorausgesetzt, dass etwa verwendete Hilfsenzyme in diesem pH-Bereich ebenfalls aktiv sind. Bevorzugt wird die Bestimmung in gepufferter Lösung bei pH-Werten von 6,5 bis 8 durchgeführt.

Wie bereits erwähnt, sind prinzipiell alle bekannten Varianten der Cholesterinbestimmung unter Verwendung von Cholesterinoxidase im Rahmen der Erfindung anwendbar. Lediglich die Messung der Cholestenonbildung bei 230 bis 250 nm eignet sich bei höheren 3,4-Dichlorphenol-Konzentrationen weniger, da 3,4-Dichlorphenol bei der Messstrahlung des Cholestenons absorbiert. Bei dieser Ausführungsform des erfindungsgemässen Verfahrens sollte daher eine Konzentration von 1,5 mmol/l 3,4-Dichlorphenol nicht überschritten werden.

Die bevorzugte Ausführungsform des erfindungsgemässen Verfahrens besteht in der Bestimmung von gebildetem Wasserstoffperoxid durch Zusatz von 4-Aminoantipyrin, Phenol, Peroxidase und einer für dieses System geeigneten Puffersubstanz. Bevorzugt werden hier Phosphatpuffer, Hepespuffer (HEPES: 4-(2-Hydroxyäthyl)-1-piperazinäthan-sulfonsäure) oder Trispuffer.

Bei dieser Ausführungsform der Erfindung erhält man die besten Ergebnisse, wenn man ausserdem ein nichtionisches Detergens und gegebenenfalls zusätzlich noch ein Detergens der Cholsäuregruppe zusetzt.

Ein weiterer Gegenstand der Erfindung ist ein Reagenz zur kinetischen Bestimmung von Cholesterin, enthaltend Cholesterinoxidase und ein System zur Bestimmung von $H_2O_2$ oder von Cholestenon sowie gegebenenfalls Cholesterinesterase, welches dadurch gekennzeichnet ist, dass es zusätzlich 3,4-Dichlorphenol enthält und die Cholesterinoxidase aus einem Mikroorganismus der Gattung Streptomyces stammt.

Als System zur Bestimmung von $H_2O_2$ wird die Kombination 4-Aminoantipyrin oder ein Derivat davon, Phenol oder Phenolderivat, Puffer und Detergens, häufig als «PAP»-System bezeichnet, bevorzugt. Das PAP-System und seine Abwandlungen sind besonders deshalb vorteilhaft, weil der gebildete Farbstoff im UV bei anderer Wellenlänge absorbiert als das 3,4-Dichlorphenol und daher keine Beeinträchtigung der Empfindlichkeit durch Überlappung der Extinktion auftreten kann. Als besonders geeignet erwies sich ein nichtionisches Detergens, welches allein oder gegebenenfalls zusammen mit einem Detergens der Cholsäuregruppe verwendet wird.

Das erfindungsgemässe Reagenz enthält vorzugsweise eine Puffersubstanz, wobei alle Puffer in Betracht kommen, die im Aktivitätsbereich der beteiligten Enzyme zu puffern vermögen, vorzugsweise im pH-Bereich von 6,5 bis 8. Als besonders geeignet erwiesen sich Phosphatpuffer, Hepespuffer und Trispuffer.

Besonders bevorzugt weist das erfindungsgemässe Reagenz, welches als System zur Bestimmung von $H_2O_2$ das PAP-System enthält, folgende quantitative Zusammensetzung auf:

0,1 bis $10 \times 10^3$ U/l Cholesterinoxidase,
0,1 bis $20 \times 10^3$ U/l Cholesterinesterase,
0,1 bis $5 \times 10^3$ U/l Peroxidase,

1 bis 10 mmol/l 3,4-Dichlorphenol,
0,5 bis 10 mmol/l 4-Aminoantipyrin,
5 bis 20 mmol/l Phenol oder Phenolderivat,
1 bis 10 g/l nichtionisches Detergens,
0 bis 15 mmol/l Detergens der Cholsäuregruppe und
50 bis 200 mmol/l Puffer, pH 6,5 bis 8.

Daneben kann das erfindungsgemässe Reagenz noch übliche Zusatzstoffe für enzymatische Reagenzien, wie Stabilisierungsmittel, z.B. Mannit, Bakterizide, wie Azid, anorganische Salze, enthalten.

Im oben erwähnten PAP-System nach Trinder können anstelle von Phenol Phenolderivate, Anilinderivate, Naphthol, Naphtholderivate, Naphthylamin, Naphthylaminiderivate. Aminochinoline, Hydroxychinoline, Dihydroxyphenylessigsäure und ähnlich reagierende Substanzen eingesetzt werden. Das 4-Aminoantipyrin kann ersetzt werden, z.B. durch Phenylendiamin-sulfonsäure, Methylbenzothiazolon-hydrazon, sulfoniertes Methylbenzothiazolon-hydrazon-Derivate und ähnlich gebaute Verbindungen.

Anstelle des PAP-Systems lassen sich jedoch im Rahmen der Erfindung auch andere bekannte $H_2O_2$-Bestimmungssysteme einsetzen. Unter diesen werden die Lumineszenzmethoden, bei denen die Fluoreszenz oder Chemilumineszenz gemessen wird, bevorzugt, beispielsweise das Peroxidase/Luminol-System. Andere geeignete Systeme bestehen aus Katalase, β-Diketonen, wie z.B. Acetylaceton, und einem Alkohol, wie Methanol, Äthanol oder Methylenglykol. Ein weiteres geeignetes System besteht aus Peroxidase und einem Chromophor, wie 2,3′-Aminobenzthiazolin-sulfonsäure.

Wenn das erfindungsgemässe Reagenz ein System zur Bestimmung von Cholestenon enthält, so eignen sich hierfür die bekannten Nachweissysteme. Wahlweise kann, wie oben bereits erwähnt, auch die Cholestenonbildung bei 240 nm direkt gemessen werden, wenn ein gewisser Empfindlichkeitsverlust in Kauf genommen wird, da bei dieser Wellenlänge auch das 3,4-Dichlorphenol schon absorbiert.

Das erfindungsgemässe Reagenz kann auch auf einem festen Träger imprägniert vorliegen. In diesem Fall kann man die Bestimmung durchführen, indem man entweder

a) den mit dem Reagenz imprägnierten Träger in die Probe eintaucht, oder

b) eine definierte Probenmenge auf den Reagenzträger auftropft oder

c) mit einem definierten Probevolumen das Reagenz vom Träger eluiert.

Wird die Reaktionsgeschwindigkeit direkt auf dem Träger gemessen, so ist dies beispielsweise reflektometrisch oder durch Lumineszenzmessung möglich. Erfolgt die Bestimmung nach Elution des Reagenz, so erfolgt die Messung wie beim trägerfreien Reagenz.

Als Trägermaterialien eignen sich die für analytische Nachweisreagenzien üblichen Träger, wie Papier, Zellulose, Faservlies, poröse Kunststoffmembranen und dergleichen. Die Herstellung erfolgt durch Eintauchen oder Besprühen mit dem erfindungsgemässen Reagenz.

Das erfindungsgemässe Verfahren erlaubt eine wesentlich schnellere Erfassung der Cholesterinmenge und führt zu einer grossen Zeitersparnis gegenüber den herkömmlichen Analysenverfahren. Für automatisierte Analysensysteme wird in vielen Fällen hierdurch die enzymatische Cholesterinbestimmung überhaupt erst praktikabel.

Die folgenden Beispiele erläutern die Erfindung.

Beispiel 1
Es wurde folgendes Reagenz verwendet:

500 U/l Cholesterinoxidase aus Streptomyces,
1500 U/l Peroxidase,
800 U/l Cholesterinesterase,
5 mmol/l 3,4-Dichlorphenol,
5 mmol/l Phenol,
1 mmol/l 4-Aminophenazon,
4,8 g/l nichtionisches Detergens (Genapol OX 100)
9,4 mmol/l Natriumdesoxycholat,
200 mmol/l Phosphatpuffer, pH 7,8.

Auf einem handelsüblichen Analysenautomaten (im Handel erhältlich unter der Bezeichnung CENTRIFICHEM 400) wird bei folgender Einstellung die Cholesterinbestimmung im Serum durchgeführt:

Temperatur: 25°C; Filter: 500 nm; $t_o$-time-delay: 60 Sekunden; $\Delta_t$-Intervall: 1 Minute; Blanke, Auto; Testmode: Term; Printout: Konz.; Standard Value: Sollwert Precilip [R]; Testcode: oo; Reagenzvolumen: 350 µl; Probe: 5 µl; Probe und Wasser: 30 µl.

Die Bestimmung ergab bis mindestens 13 mmol/l einen linearen Kurvenverlauf.

Die verwendete Cholesterinoxidase wurde aus Streptomyces griseofuscus DSM 40191 gewonnen.

Beispiel 2
Es wurde folgendes Reagenz verwendet:

500 U/l Cholesterinoxidase aus Streptomyces,
1500 U/l Cholesterinesterase,
1500 U/l Peroxidase,
5 mmol/l 3,4-Dichlorphenol,
5 mmol/l Phenol,
1 mmol/l 4-Aminophenazon,
100 mmol/l Magnesiumsulfat,
4 g/l Isotridecyläther,
100 mmol/l Tris-Puffer, pH 7,7.

Das Reagenz wurde wie in Beispiel 1 beschrieben an einem handelsüblichen Analysenautomaten (CENTRIFICHEM 400) zur Bestimmung von Cholesterin im Serum eingesetzt. Auch hier wurde bis mindestens 13 mmol/l ein linearer Verlauf festgestellt.

Analoge Ergebnisse wurden an einem anderen Analysenautomaten (Eppendorff ACP 5040) erzielt. In diesem Falle wurde ein Startreagenz mit 5 U/ml Cholesterinoxidase verwendet.

Beispiel 3
Es wurde folgendes Reagenz verwendet:

500 U/l Cholesterinoxidase aus Streptomyces,
1500 U/l Cholesterinesterase,
1500 U/l Peroxidase,
5 mmol/l 3,4-Dichlorphenol,
14 mmol/l Salicylalkohol,
0,5 mmol/l 4-Aminophenazon,
4 g/l Isotridecyläther,
10 mmol/l Natriumcholat,
50 mmol/l Hepes-Puffer, pH 6,9.

Mit diesem Reagenz wurden analoge Ergebnisse wie in den Beispielen 1 und 2 erhalten. Die Präzision in der Serie, die auf den beiden oben genannten Analysenautomaten erzielt wurde, zeigt nachstehende Tabelle.

Tabelle

| CENTRIFICHEM 400 | ACP 5040 |
|---|---|
| n = 8 | n = 8 |
| x̄ = 311,3 | x̄ = 285,6 |
| S = 6,84 | S = 2,13 |
| VK = 2,20% | VK = 0,8% |

Beispiel 4
Kinetische Bestimmung unter Messung des Cholestenons bei 240 nm
Reagenz:

| | |
|---|---|
| Tris-Puffer | 0,1 mol/l, pH 7,6 |
| Isotridecyläther | 3 g/l |
| 3,4-Dichlorphenol | 1,0 mmol/l |
| Na-Cholat | 10,0 mmol/l |
| Cholesterinoxidase | 0,2 U/ml |

Ansatz: 2 ml Reagenz, 10 µl Probe (Cholesterin-Standard)

Testbedingungen:

| | |
|---|---|
| T | 25°C |
| $E_1$ | 1 Min. nach Start der Reaktion |
| $E_2$ | 2 Min. nach Start der Reaktion |

Ergebnis:

| Cholesterinkonzentration | Extinktionsänderung |
|---|---|
| 2,59 mmol/l | 30 mE |
| 5,17 mmol/l | 60 mE |
| 10,34 mmol/l | 120 mE |

Beispiel 5

Messung des Sauerstoffverbrauchs

Reagenz:

| | |
|---|---|
| Tris-Puffer | 0,1 mol/l, pH 7,6 |
| Isotridecyläther | 3 g/l |
| 3,4-Dichlorphenol | 3 mmol/l |
| Natriumcholat | 10 mmol/l |
| Cholesterinoxidase | 2 U/ml |

Ansatz: 1,5 ml Reagenz, 10 µl Probe
Testbedingungen: T: 25°C

Die Geschwindigkeit des Sauerstoffverbrauchs wird zwischen der 2. und 3. Minute gemessen.

Ergebnis:

| Cholesterin-konzentration | relative Reaktions-geschwindigkeit |
| --- | --- |
| 2,59 mmol/l | 25 |
| 5,17 mmol/l | 50 |
| 10,34 mmol/l | 100 |

## Patentansprüche

1. Verfahren zur Bestimmung von Cholesterin in freier oder gebundener Form mittels Cholesterin-Oxidase und gegebenenfalls Cholesterin-Esterase durch Messung des Sauerstoffverbrauches, des gebildeten Wasserstoff-Peroxids oder des gebildeten Cholestenons, dadurch gekennzeichnet, dass die Bestimmung kinetisch durchgeführt wird unter Verwendung einer Cholesterin-Oxidase, die aus einem Mikroorganismus der Gattung Streptomyces gewonnen wurde und unter Zusatz von 3,4-Dichlorphenol.

2. Verfahren nach Anspruch 1, dadurch gekennzeichnet, dass man 0,5–50 mmol/l 3,4-Dichlorphenol und $0,1–50 \times 10^3$ U/l Cholesterin-Oxidase zusetzt.

3. Verfahren nach Anspruch 1 oder 2, dadurch gekennzeichnet, dass man wenigstens zwei Messungen in einem vorher bestimmten Zeitintervall vornimmt.

4. Verfahren nach einem der vorhergehenden Ansprüche, dadurch gekennzeichnet, dass man die Reaktion in gepufferter Lösung bei einem pH-Wert von 6,5–8 durchführt.

5. Verfahren nach einem der vorhergehenden Ansprüche, dadurch gekennzeichnet, dass man zur Bestimmung des gebildeten Wasserstoff-Peroxids 4-Aminoantipyrin, Phenol, Peroxidase und Phosphat-Puffer, Hepes-Puffer oder Tris-Puffer zusetzt.

6. Verfahren nach einem der vorhergehenden Ansprüche, dadurch gekennzeichnet, dass man ein nichtionisches Detergens und gegebenenfalls zusätzlich ein Detergens der Cholsäure-Gruppe zusetzt.

7. Reagenz zur kinetischen Bestimmung von Cholesterin, enthaltend Cholesterin-Oxidase und ein System zur Bestimmung von $H_2O_2$ oder von Cholestenon sowie gegebenenfalls Cholesterin-Esterase, dadurch gekennzeichnet, dass es zusätzlich 3,4-Dichlorphenol enthält und die Cholesterin-Oxidase aus einem Mikroorganismus der Gattung Streptomyces stammt.

8. Reagenz nach Anspruch 7, dadurch gekennzeichnet, dass das System zur Bestimmung von $H_2O_2$ 4-Aminoantipyrin, Phenol oder ein Derivat davon, Puffer und Detergens enthält.

9. Reagenz nach Anspruch 7 oder 8, dadurch gekennzeichnet, dass es ein nichtionisches Detergens allein oder zusammen mit einem Detergens der Cholsäuregruppe enthält.

10. Reagenz nach einem der Ansprüche 7 bis 9, dadurch gekennzeichnet, dass es Phosphat-Puffer, Hepes-Puffer oder Tris-Puffer enthält.

11. Reagenz nach einem der Ansprüche 8–10, dadurch gekennzeichnet, dass es

$0,1–10 \times 10^3$ U/l Cholesterin-Oxidase
$0,1–20 \times 10^3$ U/l Cholesterin-Esterase
$0,1–5 \times 10^3$ U/l Peroxidase
  1–10 mmol/l 3,4-Dichlorphenol
0,5–10 mmol/l 4-Aminoantipyrin
  5–20 mmol/l Phenol oder Phenol-Derivat
  1–10 gl/l nichtionisches Detergens
  0–15 mmol/l Detergens der Cholsäuregruppe
50–200 mmol/l Puffer, pH 6,5–8
enthält.

12. Reagenz nach einem der Ansprüche 7–11, dadurch gekennzeichnet, dass es auf einem festen Träger imprägniert vorliegt.

## Revendications

1. Procédé de dosage du cholestérol sous forme libre ou liée au moyen de cholestérol-oxydase et le cas échéant de cholestérol-estérase par mesure de la consommation d'oxygène, du peroxyde d'hydrogène formé ou de la cholesténone formée, caractérisé en ce que le dosage est effectué cinétiquement en utilisant une cholestérol-oxydase qui a été obtenue à partir d'un microorganisme du genre Streptomyces et avec addition de 3,4-dichlorophénol.

2. Procédé suivant la revendication 1, caractérisé en ce qu'on ajoute 0,5–50 mmole/l de 3,4-dichlorophénol et $0,1–50 \times 10^3$ U/l de cholestérol-oxydase.

3. Procédé suivant la revendication 1 ou 2, caractérisé en ce qu'on effectue au moins deux mesures dans un intervalle de temps déterminé à l'avance.

4. Procédé suivant l'une des revendications précédentes, caractérisé en ce qu'on effectue la réaction en solution tamponnée à un pH de 6,5 à 8.

5. Procédé suivant l'une des revendications précédentes, caractérisé en ce qu'on ajoute pour le dosage du peroxyde d'hydrogène formé de la 4-aminoantipyrine, du phénol, de la peroxydase et du tampon phosphate, du tampon Hepes ou du tampon tris.

6. Procédé suivant l'une des revendications précédentes, caractérisé en ce qu'on ajoute un détergent non ionique et le cas échéant en outre un détergent du groupe de l'acide cholique.

7. Réactif pour le dosage cinétique du cholestérol, contenant de la cholestérol-oxydase et un système pour le dosage de $H_2O_2$ ou de la cholesténone ainsi que, le cas échéant, de la cholestérol-estérase, caractérisé en ce qu'il contient en outre du 3,4-dichlorophénol et en ce que la cholestérol-oxydase provient d'un microorganisme du genre streptomyces.

8. Réactif suivant la revendication 7, caractérisé en ce que le système pour le dosage de $H_2O_2$ contient de la 4-aminoantipyrine, du phénol ou un de ses dérivés, un tampon et un détergent.

9. Réactif suivant la revendication 7 ou 8, caractérisé en ce qu'il contient un détergent non ionique seul ou en même temps qu'un détergent du groupe de l'acide cholique.

10. Réactif suivant l'une des revendications 7 à 9, caractérisé en ce qu'il contient un tampon phosphate, du tampon Hepes ou du tampon tris.

11. Réactif suivant l'une des revendications 8–10, caractérisé en ce qu'il contient:

0,1–10 × 10³ U/l de cholestérol-oxydase,
0,1–20 × 10³ U/l de cholestérol-estérase,
0,1– 5 × 10³ U/l de peroxydase,
1–10 mmole/l de 3,4-dichlorophénol,
0,5–10 mmole/l de 4-aminoantipyrine,
5–20 mmole/l de phénol ou d'un dérivé du phénol,
1–10 g/l de détergent non ionique,
0–15 mmole/l d'un détergent du groupe de l'acide cholique,
50–200 mmole/l de tampon, pH 6,5–8.

12. Réactif suivant l'une des revendications 7–11, caractérisé en ce qu'il est à l'état imprégné sur un support solide.

**Patent Claims**

1. Process for the determination of cholesterol in free or bound form by means of cholesterol oxidase and possibly of cholesterol esterase by measurement of the oxygen consumption, of the hydrogen peroxide formed or of the cholestenone formed, characterised in that the determination is carried out kinetically with the use of a cholesterol oxidase which has been obtained from a micro-organism of the genus Streptomyces and with the addition of 3,4-dichlorophenol.

2. Process according to claim 1, characterised in that one adds 0.5–50 mmole/l. 3,4-dichlorophenol and 0.1–50 × 10³ U/l. cholesterol oxidase.

3. Process according to claim 1 or 2, characterised in that one carries out at least two measurements in a predetermined interval of time.

4. Process according to one of the preceding claims, characterised in that one carries out the reaction in buffered solution at a pH value of 6.5–8.

5. Process according to one of the preceding claims, characterised in that, for the determination of the hydrogen peroxide formed, one adds 4-aminoantipyrine, phenol, peroxidase and phosphate buffer, hepes buffer or tris buffer.

6. Process according to one of the preceding claims, characterised in that one adds a non-ionic detergent and possibly additionally a detergent of the cholic acid group.

7. Reagent for the kinetic determination of cholesterol, containing cholesterol oxidase and a system for the determination of $H_2O_2$ or of cholestenone, as well as possibly cholesterol esterase, characterised in that it additionally contains 3,4-dichlorophenol and the cholesterol oxidase originates from a micro-organism of the genus Streptomyces.

8. Reagent according to claim 7, characterised in that the system for the determination of $H_2O_2$ contains 4-aminoantipyrine, phenol or a derivative thereof, buffer and detergent.

9. Reagent according to claim 7 or 8, characterised in that it contains a non-ionic detergent alone or together with a detergent of the cholic acid group.

10. Reagent according to one of claims 7 to 9, characterised in that it contains phosphate buffer, hepes buffer or tris buffer.

11. Reagent according to one of claims 8 to 10, characterised in that it contains

0.1–10 × 10³ U/l. cholesterol oxidase
0.1–20 × 10³ U/l. cholesterol esterase
0.1–5 × 10³ U/l. peroxidase
1–10 mmol/l. 3,4-dichlorophenol
0.5–10 mmol/l. 4-aminoantipyrine
5–20 mmol/l. phenol or phenol derivative
1–10 g/l. non-ionic detergent
0–15 mmol/l. detergent of the cholic acid group
50–200 mmol/l. buffer, pH 6.5–8.

12. Reagent according to one of claims 7 to 11, characterised in that it is present impregnated on a solid carrier.